# EUROPEAN PATENT SPECIFICATION

(11) **EP 2 920 323 B1**
(45) Date of publication and mention of the grant of the patent: **25.04.2018**
(21) Application number: 13798507.3
(22) Date of filing: 15.11.2013
(51) Int. Cl.: C12N 15/10, C12Q 1/68

(54) **METHOD FOR PROCESSING PARAFFIN EMBEDDED SAMPLES**
VERFAHREN ZUR VERARBEITUNG VON IN PARAFFIN EINGEBETTETEN PROBEN
PROCÉDÉ POUR TRAITER DES ÉCHANTILLONS INCLUS DANS LA PARAFFINE

(30) Priority: 16.11.2012 US 201213678755
(43) Date of publication of application: 23.09.2015
(73) Proprietor: Covaris, Inc., Woburn, MA 01801-1721 (US)
(72) Inventor: LAUGHARN, James, A., Jr., Winchester, MA 01890 (US); RUDD, Edwin, Salem, NH 03079 (US); DURIN, Guillaume, 69007 Lyon (FR)
(74) Representative: Hoffmann Eitle
(86) International application number: PCT/US2013/070290
(87) International publication number: WO 2014/078650

(56) References cited:
- WO-A2-2005/116256
- COOMBS N J ET AL: "Optimisation of DNA and RNA extraction from archival formalin-fixed tissue", NUCLEIC ACIDS RESEARCH, OXFORD UNIVERSITY PRESS, GB, vol. 27, no. 16, 15 August 1999 (1999-08-15), pages e12/1-E12/3, XP002242203, ISSN: 0305-1048, DOI: 10.1093/NAR/27.16.E12
- BANERJEE S K ET AL: "MICROWAVE-BASED DNA EXTRACTION FROM PARAFFIN-EMBEDDED TISSUE FOR PCR AMPLIFICATION", BIOTECHNIQUES, INFORMA HEALTHCARE, US, vol. 18, no. 5, 1 January 1995 (1995-01-01), XP009011206, ISSN: 0736-6205
- WU SHANSHUI ET AL: "Microwave heating of long-term formalin-fixed surgical pathology specimens improves quality of extracted DNA", APPLIED IMMUNOHISTOCHEMISTRY & MOLECULAR MORPHOLOGY: AIMM / OFFICIAL PUBLICATION OF THE SOCIETY FOR APPLIED IMMUNOHISTOCHEMISTRY,, vol. 20, no. 5, 1 October 2012 (2012-10-01), pages 512-517, XP009175473, ISSN: 1533-4058, DOI: 10.1097/PAI.0B013E3182434174
- FRANK T S ET AL: "Comparison of methods for extracting DNA from formalin-fixed paraffin sections for nonisotopic PCR", DIAGNOSTIC MOLECULAR PATHOLOGY, LIPPINCOTT WILLIAMS AND WILKINS, US, vol. 5, no. 3, 1 September 1996 (1996-09-01), pages 220-224, XP009175489, ISSN: 1052-9551, DOI: 10.1097/00019606-199609000-00012

## Description

### 1. Field of the Invention

The present invention relates to a method for processing a paraffin-embedded sample, although systems for performing the method are also disclosed..

### 2. Related Art

Tissue samples, such as those taken by biopsy or other technique, are commonly formalin-fixed and paraffin embedded (FFPE) to allow for extended storage of the samples. Ideally, the extended storage maintains the integrity of the sample such that the ability for subsequent analysis, for example, histopathology and nucleic acid based diagnostics, is preserved, with relatively little degradation of DNA, RNA, proteins or other materials in the sample. In such FFPE processing, the samples are typically fixed in a formalin solution (e.g., a 10% formalin solution may contain 3.7% formaldehyde and 1.0 to 1.5% methanol), which creates crosslinks between nucleic acids, between proteins and/or between nucleic acids and proteins. Afterward, the sample is dehydrated, e.g., by placing the sample in an alcohol, and then "cleared" of the alcohol by exposing the sample to a solvent such as xylene. The sample is then embedded in paraffin, where the sample is surrounded by paraffin which replaces the xylene in the sample. The paraffin embedded sample can then be stored for extended periods of days, months, years.

To recover nucleic acid material (e.g., DNA and/or RNA) and/or proteomic material (e.g., proteins) from an FFPE sample, the paraffin must be disassociated from the sample. This is typically done by placing the paraffin-bearing sample in an organic solvent, such as xylene, heptane or limonene, to dissolve the paraffin or by heating the sample in mineral oil or aqueous buffer.

WO 2005/116256 A2 describes a device for extracting biological molecules from a tissue specimen comprising: a base having a top surface and a bottom surface, wherein said top surface of said base holds said tissue specimen; a slide cover adapted to mount on said top surface of said base, wherein said slide cover has an inner surface, wherein said top surface of said base and said inner surface of said slide cover form a space for retaining an extraction buffer; and a thermal control device adapted to be connected to said bottom surface of said base.

Coombs et al. have reported a study whereby they examine different methods to extract DNA from paraffin-embedded samples (Nucleic Acids Res. 1999, 27(16), pp. e12(i)-(iii)). These methods include placing the paraffin-bearing sample in xylene/ethanol, heating the sample, and irradiating the sample with microwave radiation.

Microwave-based DNA extraction of paraffin-embedded tissue has also been reported by Banerjee et al. (Biotechniques, 1995, 18(5), pp. 768-770) and Wu et al. (App. Immunohistochemistry & Mol. Morphology, 2012, 20(5), pp. 512-517).

Frank et al. also describe a study where different methods are used to extract DNA from paraffin-embedded samples (Diagnostic Molecular Pathology, 1996, 5(3), pp. 220-224), including proteinase digestion with and without detergents, as well as dewaxing in xylene or by boiling.

### SUMMARY OF INVENTION

The present invention relates to a method for processing a paraffin-embedded sample, comprising:
providing a paraffin-embedded tissue sample in a vessel, the sample having previously been formalin fixed and embedded in paraffin;
providing a solution in the vessel with the paraffin-embedded sample, wherein the solution is a non-solvent solution for the paraffin;
disassociating paraffin from the paraffin-embedded sample by exposing the sample and non-solvent solution in the vessel to acoustic energy having a frequency of between 100 kilohertz and 100 megahertz and a focal zone with a width of less than 2 centimeters to disassociate paraffin from the sample; and
recovering biomolecules from the sample after disassociation of paraffin from the sample.

The inventors have found that exposing the sample to a solvent during paraffin removal tends to cause damage to the nucleic acid and/or proteome material or otherwise tends to reduce the yield of good quality nucleic acid and/or proteomic material recovered from an FFPE sample. In addition, the sample is now a hazardous organic solvent waste and proper disposal is more problematic. In accordance with one aspect of the invention, paraffin may be disassociated from an FFPE sample using a non-solvent solution, e.g., without exposing the sample to a solvent during the process of paraffin disassociation. Instead, a non-solvent solution, e.g., one that includes water and a detergent, may be used together with suitable focused acoustic energy to disassociate paraffin from a sample. Such paraffin disassociation may be done without exposing the sample to relatively high temperatures, e.g., paraffin may be suitably disassociated from the sample while maintaining the sample temperature below 5-60 degrees C (e.g., between 1-30 degrees C, approximately 20 degrees C, approximately 7 degrees C). This paraffin disassociation technique has been found to increase nucleic acid material yield by at least 2 to 4 times that found with typical processes. In some embodiments, paraffin may be disassociated from a sample relatively quickly, e.g., in 3 minutes or less. Also, in some embodiments the sample may be rehydrated during the paraffin disassociation process, which has been found to improve bio-material yield as well.

In another aspect of the invention, and within the scope of the method defined by the appended claims, the digesting of crosslink materials in preparation for nucleic acid material purification can be enhanced. In one embodiment, a FFPE sample may be exposed to a proteinase K enzyme or other protease along with suitable focused acoustic energy for a relatively short period, e.g., 30 seconds or less, to help enhance release of nucleic acid material by digesting crosslinks caused by the FFPE process. In some embodiments, the protease may be combined with a glycerol to enhance enzyme activity during acoustic treatment.

In one aspect of the invention, and within the scope of the method defined by the appended claims, a method for processing a paraffin-embedded sample includes providing a paraffin-embedded tissue sample in a vessel, where the sample has previously been formalin fixed and embedded in paraffin and has paraffin attached to the sample. A non-solvent, aqueous solution is provided in the vessel with the paraffin-embedded sample, and paraffin is disassociated from the paraffin-embedded sample by exposing the sample and non-solvent solution in the vessel to acoustic energy to disassociate paraffin from the sample. Biomolecules, such as nucleic acids, proteins and/or other components, may be recovered from the aqueous portion of the sample after disassociation of paraffin, e.g., by pipetting liquid containing the biomolecules from the vessel. If desired, the sample may be subject to further focused acoustic treatment, for example, for additional processing (e.g., fragmenting of nucleic acids) and/or to enhance overall recovery of biomolecules.

The disassociation process may include exposing the sample to focused acoustic energy for a time sufficient to disassociate enough paraffin from the sample to allow recovery of nucleic and/or proteome material from the sample. For example, 90%, 95%, 98% or more of the paraffin initially attached to the sample may be disassociated from the sample, e.g., by emulsifying the paraffin. Since the liquid in the vessel is aqueous, disassociation of the paraffin may also include rehydrating the sample while exposing the sample to focused acoustic energy. Disassociation may performed while the vessel is located in a bath of liquid at a temperature of about 5-60 degrees C, e.g., the bath may be at a temperature of about 40 degrees C, or a temperature of about 20 degrees C, or a temperature of about 7 degrees C. Thus, disassociation may be performed while the temperature of the sample is maintained below about 60 degrees C, e.g., below about 45 degrees C, below about 20 degrees C, below about 10 degrees C.

Recovery of the biomolecules may include a variety of different processes, such as adding a protease, or other enzyme (e.g., DNase, RNase), to the non-solvent aqueous solution and the sample in the vessel after disassociation of paraffin from the sample. The processed sample and an enzyme-containing solution may be exposed to focused acoustic energy a second time, e.g., for a period of 10-30 seconds (or more) to enhance the mixing of the enzyme with the sample and thereby enhance enzymatic activity.

The mixed sample is then incubated with the enzyme to digest the tissue for suitable period of time (e.g., 15 minutes, 1 hour, 2 hours, 3 hours) at an appropriate temperature. Crosslinks in the sample material may be reversed by an additional incubation at 80 - 90 degrees C for an extended period, e.g., for 1 hour or more, or less than 1 hour (e.g., 15 minutes). The sample may also be exposed to focused acoustic energy suitable to shear nucleic acid material released from the sample into smaller fragments. For example, a majority of the fragments of nucleic acid material after exposing the sample to focused acoustic energy may have a size of 50 to 1000 bp. The nucleic acid fragment size can be tuned according to the parameters under which the focused acoustic energy is generated. Enzyme treatment and/or nucleic acid fragmentation may be done in the vessel containing the disassociated paraffin and the sample as part of the recovering step. After reversal of the crosslinks, the sample may be subject to a short burst of focused acoustic energy, resulting in a greater nucleic acid recovery.

Also disclosed herein, although not claimed, is an acoustic treatment device that includes a vessel holding a formalin fixed, paraffin embedded tissue sample and a non-solvent, aqueous solution, and an acoustic energy source for providing acoustic energy to the sample while the sample is in the vessel and separated from the acoustic energy source. A vessel holder may support the vessel at a location at least partially in a focal zone of the acoustic energy, and a system control circuit may control the acoustic energy source to expose the sample to focused acoustic energy suitable to disassociate paraffin from the sample to allow recovery of biomolecules of the sample. In some embodiments, the acoustic energy source is spaced from and exterior to the vessel, and the acoustic energy comprises a frequency of between about 100 kilohertz and about 100 megahertz and a focal zone having a width of less than about 2 centimeters, and wherein at least a portion of the acoustic energy is adapted to propagate exterior to the vessel. In addition, by combining paraffin disassociation, tissue sample rehydration, enzyme mixing, and tissue sample digestion into one vessel the process may be readily automated. For example, if the vessel includes a cap with a split septa, an enzyme (e.g., protease, DNase, RNase, etc.) may be added following the disassociation of the paraffin without removal of the cap.

Other advantages and novel features of the invention will become apparent from the following detailed description of various non-limiting embodiments when considered in conjunction with the accompanying figures and claims.

### BRIEF DESCRIPTION OF THE DRAWINGS

Aspects of the invention are described with reference to the following drawings in which numerals reference like elements, and wherein:
FIG. 1 shows a schematic block diagram of an acoustic treatment system that may be used to perform the method of the invention;
FIG. 2 shows steps in a method for processing a paraffin embedded sample;
FIG. 3 depicts a perspective view of a collecting tool in accordance with some embodiments;
FIGs. 4-5 show nucleic acid size distributions for some examples;
FIG. 6 illustrates nucleic acid recovery for some examples; and
FIG. 7 shows fluorescence micrographs of tissue sections for some examples.

### DETAILED DESCRIPTION

As described above, chemical, biochemical, and acoustic treatment processes can be useful for disassociating paraffin from FFPE samples and/or digesting crosslinked proteins in the sample material with the end goal of recovering target molecules from the sample material, such as DNA, RNA, proteins, and the like. In addition, such systems may be used along with aspects of the invention for DNA/RNA shearing, e.g., to reduce the base pair length of DNA fragments from 1,000s or 10,000s of base pairs to lengths of 3k base pairs or smaller. Examples of such acoustic treatment systems and control arrangements are described in U.S. Patents 6,948,843 and 6,719,449, assigned to Covaris of Woburn, Mass.

In some embodiments, and within the scope of the method defined by the appended claims, aspects of the invention may allow for paraffin disassociation from a FFPE sample, protease treatment or other processing to digest unwanted material to release DNA/RNA from the sample, and/or shearing to fragment nucleic acid material into a desired size range all in a single vessel using a non-solvent aqueous solution and without the use of solvents. This series of steps performed in a single vessel (e.g., Covaris microTUBE) and without the use of solvents is simply not possible with conventional techniques, and allows for improved nucleic acid material yield and decreased possibility for sample contamination.

In another aspect of the invention, and within the scope of the method defined by the appended claims, an efficiency of use of acoustic energy in disassociating paraffin from the sample, enhancing activity of an enzyme and/or fragmentation of nucleic acid material may be enhanced, e.g., by reducing an amount of gas that is entrained in the sample or available for entrainment. In some embodiments, gas entrainment in a sample may be reduced by reducing or otherwise controlling a size of the headspace adjacent the sample. By reducing the volume and/or surface area of a headspace presented to a sample, an amount of gas available for entrainment into the sample can be reduced, or a rate at which the gas can be entrained may be reduced. This can help reduce bubble formation in the sample during acoustic treatment and/or interference of gas with cavitation bubble collapse, helping to increase an amount of acoustic energy that is used for treating the sample rather than being reflected out of the sample vessel or absorbed by increased cavitation bubble pressure.

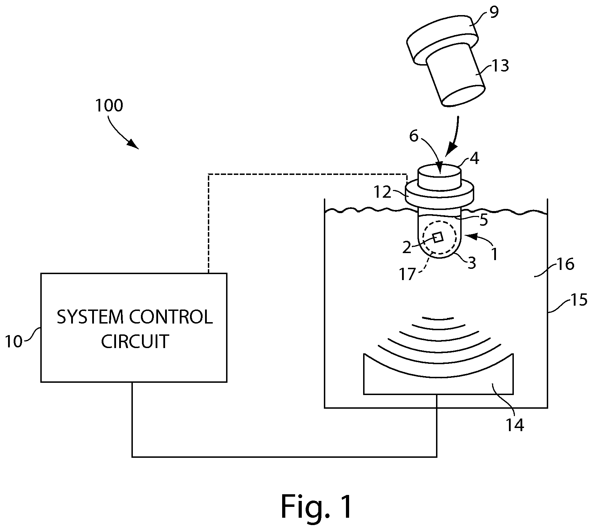

FIG. 1 shows a schematic block diagram of an acoustic treatment system 100 that may be used to perform the method of the invention. In this illustrative embodiment, the acoustic treatment system 100 includes an acoustic transducer 14 (e.g., including one or more piezoelectric elements) that is capable of generating an acoustic field (e.g., at a focal zone 17) suitable to cause mixing, e.g., caused by cavitation, and/or other effects in a sample 1 contained in a vessel 4. The acoustic transducer 14 may produce acoustic energy within a frequency range of between about 100 kilohertz and about 100 megahertz such that the focal zone 17 has a width of about 2 centimeters or less. The focal zone 17 of the acoustic energy may be any suitable shape, such as spherical, ellipsoidal, rod-shaped, or column-shaped, for example, and be positioned at the sample 1. The focal zone 17 may be larger than the sample volume, or may be smaller than the sample volume, as shown in FIG. 1, e.g., the focal zone 17 may fit entirely within the vessel 4. U.S. Patents 6,948,843 and 6,719,449 are referred to for details regarding the construction and operation of an acoustic transducer and its control.

The vessel 4 may have any suitable size or other arrangement, e.g., may be a glass tube, a plastic container, a well in a microtiter plate, a vial, or other, and may be supported at a location by a vessel holder 12. In this embodiment, the vessel 4 is a 6x16mm glass or plastic tube (approximately 150 microliter volume) having a screw cap, but it should be understood that the vessel 4 may have any suitable shape, size, material, or other feature. For example, the vessel 4 may be a cylindrical tube with a flat bottom and a threaded top end to receive a cap 9, may include a cylindrical collar with a depending flexible bag-like portion to hold a sample, may be a single well in a multiwell plate, may be a cube-shaped vessel, or may be of any other suitable arrangement. The vessel 4 may be formed of glass, plastic, metal, composites, and/or any suitable combinations of materials, and formed by any suitable process, such as molding, machining, stamping, and/or a combination of processes.

The acoustic treatment system 100 may also include a coupling medium container 15 that is capable of holding a medium 16 (such as water or other liquid, gas, gel, solid, semi-solid, and/or a combination of such components) which transmits acoustic energy from the transducer 14 to the vessel 4. In embodiments where the medium 16 includes a solid or semi-solid, a container 15 need not be provided or a portion of the medium 16 itself may function as a container 15, e.g., to hold a liquid or gas portion of the medium 16. For example, in one embodiment, the transducer 14 may be attached to a solid coupling medium 16 (such as a silica material), which is also attached to a vessel holder 12, which may be formed, at least in part, by an opening or other feature of the medium 16. Thus, the transducer 14, medium 16 and holder 12 may be formed as a single integrated part, if desired. In some embodiments, the acoustic field may be controlled, the acoustic transducer 14 may be moved, and/or the vessel 4 may be moved (e.g., by way of moving a holder 12, such as a rack, tray, platform, etc., that supports the vessel 4) so that the sample is positioned in a desired location relative to the focal zone 17. In addition, or alternately, the transducer 14 may form the focal zone 17 so that the focal zone 17 is suitably positioned relative to the sample 1 or vessel 4.

To control the acoustic transducer 14, the acoustic treatment system 100 may include a system control circuit 10 that controls various functions of the system 100 including operation of the acoustic transducer 14. For example, the system control circuit 10 may provide control signals to a load current control circuit, which controls a load current in a winding of a transformer. Based on the load current, the transformer may output a drive signal to a matching network, which is coupled to the acoustic transducer 14 and provides suitable signals for the transducer 14 to produce desired acoustic energy. As discussed in more detail below, the system control circuit 10 may control various other acoustic treatment system 100 functions, such as positioning of the vessel 4 and/or acoustic transducer 14 (a dashed line linking the control circuit 10 to the holder 12 schematically represents an optional positioning system, e.g., including a robot, gantry, screw drive, or other arrangement to move the holder 12), receiving operator input (such as commands for system operation), outputting information (e.g., to a visible display screen, indicator lights, sample treatment status information in electronic data form, and so on), and others.

In this illustrative embodiment, the sample 1 includes a solid material 2, such as a tissue sample that has been formalin fixed and paraffin embedded (i.e., an FFPE sample) that is contained in the vessel 4 along with a liquid 3, e.g., a non-solvent aqueous solution. The non-solvent solution 3 may include water along with a detergent, e.g., a 0.25% SDS (sodium dodecyl sulfate) solution, although other solutions are possible.

The sample may have any suitable volume and/or mass, e.g., the sample may be a so-called "scroll," a "core," or piece of FFPE tissue microtome sliced from a larger sample piece, a tissue sample taken by needle biopsy, or other. For example, a core of sample may be punched from a larger sheet of tissue sample. In some embodiments, a sample cut by microtome may have a thickness of about 7 to 25 micrometers and a length of 20 mm or less. The sample may have any suitable thickness, for example, between about 0.5 mm and about 20 mm (e.g., 1-10 mm or 1-5 mm in thickness). The sample may have an appropriate volume. For example, a sample may be sized to have a volume of about 4 cubic millimeters or less. Of course, depending on the application or sample involved, other volumes may be used, such as less than 10 cubic millimeters, less than 20 cubic millimeters, less than 50 cubic millimeters, less than 100 cubic millimeters, or less than 500 cubic millimeters. In some embodiments, a sample may be between about 0.5 mm and about 5 mm in diameter (e.g., 1-3 mm in diameter).

In accordance with an aspect of the invention, if using the acoustic treatment device as disclosed herein for the method defined by the appended claims, care may be taken to suitably define a headspace 6 in the vessel 4 prior to acoustic treatment. That is, an interface 5 (or upper level of the liquid 3 in the vessel 4) may be separated from the cap 9 by a headspace 6, which is shown to be a gaseous region immediately above the interface 5. By appropriately setting the headspace 6 volume, efficiency of the paraffin disassociation, protease treatment and/or nucleic acid fragmentation process may be enhanced. For example, some acoustic energy power levels at the focal zone 17 suitable to cause mixing, e.g., lysing, extraction, permeabilizing, catalyzing, degrading, fluidization, heating, particle breakdown, shearing and/or disruption of molecular bonds in the sample 1, may also cause portions of the sample 1 (including solid material 2 and/or liquid material 3) to be splashed or otherwise ejected from the interface 5. In some cases, the ejected sample 1 may return to the main volume of sample 1, but in other cases, the ejected sample 1 may adhere to the vessel 4 above the interface 5 or otherwise fail to return to the main sample 1. In either case, the ejected sample 1 may spend a reduced amount of time in the focal zone 17.

In addition, or alternately, acoustic energy may cause gas in the headspace 6 to be entrained into the liquid 3, such as by dissolving a portion of the gas in the headspace 6 and/or by capturing bubbles of headspace gas in the sample due to motion of the liquid at the interface 5. Gas in the liquid 3 may interfere with acoustic energy, such as by gas bubbles at or near the focal zone 17 reflecting acoustic energy away from the sample 1 and/or by dissolved gas increasing a pressure in cavitation bubbles created by acoustic energy, thereby decreasing the rate or force at which the cavitation bubbles collapse. It is believed that the collapse of cavitation bubbles transfers significant kinetic energy to sample materials, causing the materials to be lysed, sheared or otherwise mechanically operated on. By increasing a pressure in such bubbles, dissolved gas in the sample can reduce the energy released by cavitation bubble collapse, reducing an effectiveness of acoustic treatment. Thus, by controlling headspace size (volume and/or surface area presented at the interface 5), efficiency of the acoustic treatment processing can be improved. In this illustrative embodiment, liquid 3 may be provided in the vessel so that the interface 5 is within about 1-2mm of the cap 9. However, other headspace sizes are possible, including a headspace that is 20% of the liquid 3 volume, 10% of the liquid volume, 5% of the liquid volume, or 0% of the liquid volume. As shown in FIG. 1, the cap 9 may have a headspace control member 13, such as a lower portion of the cap 9 that is controllably positionable relative to the interface 5, or the headspace 6 may be controlled by suitably filling the vessel 4. The cap 9 may also include a metal or ceramic component (e.g., a disc about 1mm thick) or other relatively hard surface positioned adjacent the interface 5 to help reflect acoustic energy back toward the sample 1. This may help enhance efficiency of acoustic processing.

With the sample 1 and liquid 3 in the vessel 4, the vessel 4 may be associated with a holder 12 that helps support the vessel 4 during acoustic treatment. The holder 12 may take any suitable arrangement, such as a ring-shaped element 12 that is fixed relative to the vessel 4, as shown in FIG. 1. Although in the FIG. 1 embodiment the holder 12 is located near a middle of the vessel 4, the holder 12 may be positioned in any suitable manner relative to the vessel 4, such as near the bottom or top of the vessel, extending from one side of the vessel, and/or any other appropriate position. The holder 12 may be permanently fixed to the vessel 4, e.g., molded integrally with the vessel 4, attached to the vessel 4 by an adhesive, a fastener, welding, etc., or may be removably attached to the vessel. For example, in some embodiments, the vessel holder 12 may include a ring member like that shown in FIG. 1 and one or more O-rings (not shown) or other friction-enhancing elements that are positioned between the ring member and the vessel 4 to provide a tight friction fit between the vessel 4 and the holder 12. Such an arrangement may be useful when interchanging vessels 4 on a single holder 12 and/or adjusting the position of the vessel 4 relative to the holder 12.

Although a vessel holder 12 is not necessarily required, the vessel holder 12 may serve to interface with the acoustic processing device so that the vessel 4 and the sample in the vessel is positioned in a known location relative to an acoustic field, for example, at least partially within a focal zone 17 of acoustic energy. Also, the holder 12 is not limited to a device like that shown in FIG. 1, and instead may include a rack, slot, tray, gripper element, clamp, box or any other suitable arrangement for holding and/or moving the vessel 4 with respect to the focal zone 17.

With the vessel 4 and sample 1 suitably positioned relative to the acoustic transducer 14, acoustic treatment of the sample 1 may be performed to disassociate paraffin from the sample 1. During this process the coupling medium 16 may be maintained at a relatively low temperature, e.g., 5-60 degrees C, although lower or higher temperatures are possible. Thus, the sample 1 may be maintained at a relatively low temperature during paraffin disassociation, e.g., the sample 1 may not exceed a temperature of 5-60 degrees C during processing, and in some cases may remain below a melting temperature of the paraffin. As a result, paraffin may be disassociated from sample material without causing bulk melting of the paraffin. In an embodiment where the acoustic treatment system 100 is a Covaris S220 or E220 model, acoustic treatment may be applied using a 10% duty factor, a peak incident power of 175 watts, 200 cycles per burst for about 150-450 seconds (e.g., 300 seconds). Of course, other duty cycles, peak power, cycles per burst and/or time periods may be used. For example, the acoustic treatment system may generate focused acoustic energy according to any suitable set of parameters, such as 10-30% duty cycle, 50-450 watt peak incident power, 100-300 cycles per burst, for a suitable period of time (e.g., between 10-450 seconds). It can be appreciated that focused acoustic parameters outside the above noted ranges may be employed.

The coupling medium 16, which may be water, may be kept at a temperature of 46 degrees C, 20 degrees C, 7 degrees C, or another suitable temperature, during the paraffin disassociation processing. Also, since the liquid is a non-solvent, aqueous solution, the sample 1 may be rehydrated during paraffin disassociation. While exposing the sample to focused acoustic energy during the disassociation process, the liquid 3 will tend to appear opalescent (e.g., milky white) as the paraffin is emulsified or otherwise separated from tissue portions of the sample. This appearance is due to the disassociation of paraffin into tiny droplets (e.g., approximately 0.1-10 microns in diameter) that have been stripped from the tissue sample.

Upon completion of the paraffin disassociation process, biomolecules or other portions of the tissue sample may be recovered from the vessel, e.g., by centrifuging and/or pipetting the sample portions from the vessel, and the recovered sample portions may be subsequently treated in another vessel or holder, e.g., to recover nucleic acid, protein or other components of the sample. In one aspect of the invention, further processing of the sample, such as protease digestion, nucleic acid fragmentation, centrifugation and/or other processes may be performed in the same vessel. Performing multiple processes, e.g., as part of nucleic acid purification protocol, in a single vessel may not only simplify the overall process, but also reduces transfer losses and the chance that the sample is contaminated in some way.

In one illustrative embodiment, after paraffin disassociation is complete, a protease, such as proteinase K or trypsin, may be added to the vessel with or without removal of the disassociated paraffin from the vessel. As is known to those of skill in the art, a protease may function to digest proteins as a precursor to recovering desired nucleic acids, protein fragments or other biomolecules. In accordance with an aspect of the invention, the vessel containing a protease may be treated with acoustic energy to enhance mixing and/or activity of the protease. In one embodiment, acoustic treatment for 30 seconds or less (e.g., 10 seconds) may serve to suitably mix the protease with the sample prior to incubating the sample with the protease to further hydrolyize the proteins in the sample. Also, the inclusion of a glycerol material with the protease is thought to further enhance the enzyme activity and the effect of the acoustic energy as a driver of the protease action. This mixing treatment may be performed with the sample at a temperature of between 5-46 degrees C, e.g., with the coupling medium 16 at about 46 degrees C, about 20 degrees C, about 7 degrees C, although other temperatures are possible.

After protein digestion, the sample may be incubated with the protease at 80-90 degrees C to reverse formaldehyde cross links, e.g., at about 80 degrees C. This incubation may be performed with or without acoustic treatment of any kind. After incubation, nucleic acids, proteins or other biomolecules may be recovered from the vessel, e.g., by centrifuging and pipetting the processed suspension from the vessel. The recovered biomolecules may be subjected to any suitable further processing as desired, such as DNA purification processing using commercially available techniques and equipment.

While biomolecules within the sample may be recovered immediately after incubation, without further acoustic treatment, for some embodiments, the sample may be subjected to an additional acoustic treatment. For example, it may be desirable for nucleic acids within the sample to be further fragmented to a desired size range. Accordingly, the starting DNA material after protease incubation and reverse crosslink may include DNA segments having a preferred size of about 7-50kbp or more. Or, for some embodiments, a quick burst of focused acoustic energy may result in greater recovery yields of the desired biomolecule(s).

To shear nucleic acid material (e.g., DNA, RNA), additional focused acoustic treatment may be provided, e.g., using a Covaris S2 AFA machine employing a 10% duty factor, 175 watts peak intensity power, and 200 cycles per burst for approximately 7-8 minutes. After treatment, a majority (if not all) of the nucleic acid fragments in the sample 1 may be reduced in size to about 200bp. That is, a fairly narrow range of final nucleic acid fragment sizes may be produced, e.g., most of the nucleic acid fragments may fall in a size range of about 50bp to about 500bp, and the range of nucleic acid fragment sizes produced may be adjusted by adjusting characteristics of the acoustic treatment.

As discussed above, after crosslink reversal (e.g., at 80-90 C), the sample may be exposed to a short period of focused acoustic energy, so as to result in an increase in overall nucleic acid recovery. For example, samples processed by acoustic energy generated from a Covaris S220 or E220 system set to a 10% duty factor, 105 Watt peak incident power and 200 cycles per burst (or, Covaris S2 or E210 systems set to a 10% duty factor and 200 cycles per burst; Covaris M220 systems set to a 20% duty factor, 75 Watt peak incident power and 200 cycles per burst; and Covaris LE220 systems set to a 30% duty factor, 300 Watt peak incident power and 200 cycles per burst), for 10 seconds at 20 degrees C may result in a greater nucleic acid yield from the sample than if no acoustic energy were provided.

Those of skill in the art will appreciate that various combinations of the processing steps described herein may be employed. For instance, as described herein, depending on the amount and processing parameters of focused acoustic energy applied to the sample after paraffin disassociation, and other steps (e.g., digestion, crosslink reversal, etc.), nucleic acids may be further fragmented, or processed in such a manner that results in an increased overall nucleic acid recovery. Accordingly, methods of extracting nucleic acids described herein may result in enhanced DNA/RNA availability, reproducibility and integration with next generation sequencing and other molecular analysis applications.

As discussed, after paraffin disassociation, enzymatic digestion and crosslink reversal, a nucleic acid sample may be further fragmented, and then extracted. For example, such fragmentation may be useful for next generation sequencing analysis. Accordingly, the nucleic acid fragment size of the sample may be tuned to any suitable range (e.g., between 200-400 bp), based on the parameters of focused acoustic treatment. For instance, to achieve a target nucleic acid size of approximately 200 bp, it may be appropriate for the sample to be exposed to focused acoustic energy generated from Covaris E- and S-Series focused ultrasonicators set to a 10% duty factor, 175 Watt peak incident power, 200 cycles per burst at 20 degrees C, for approximately 300 seconds. To achieve a target nucleic acid size of 300 bp, under the same general processing conditions, a suitable treatment time may be approximately 110 seconds; and for a target nucleic acid size of 400 bp, a suitable treatment time may be approximately 80 seconds.

In some embodiments, and as discussed, after paraffin disassociation, enzymatic digestion and crosslink reversal, the nucleic acid sample may be subject to a short burst of focused acoustic treatment for "release" of the nucleic acid, surprisingly, resulting in an enhanced nucleic acid recovery. Such a short burst may result in a greater amount of nucleic acid recovery than if no focused acoustic treatment were provided. This short burst may also result in more biomolecule recovery than if the sample were exposed to focused acoustic treatment for a longer period of time, such as the amount of acoustic treatment used to tune nucleic acid fragment sizes, as discussed above. Accordingly, for some embodiments, the sample may be subject to approximately 10 seconds of additional focused acoustic energy, generated from Covaris E220 or S-220 systems set to a 10% duty factor, 105 Watt peak incident power, 200 cycles per burst at 20 degrees C. A short burst of additional focused acoustic energy may fall within other ranges of time that are suitable to enhance biomolecule recovery yield. For example, the short burst of focused acoustic energy may occur for a time period of between 1 second and 30 seconds, between 5 seconds and 20 seconds, between 10 seconds and 15 seconds, etc. Exposure to focused acoustic energy even for a short period of time may still cause nucleic acids within the sample to be slightly fragmented, for example, to sizes larger than approximately 2.0 kbp, larger than approximately 2.5 kbp, or larger than approximately 3.0 kbp.

In some embodiments, after paraffin disassociation, enzymatic digestion and crosslink reversal, the nucleic acid sample may be extracted without further exposure to focused acoustic energy. As a result, the fragment size of the nucleic acid extracted from the sample may be larger than if the sample were exposed to any additional focused acoustic energy. For example, upon recovery, genomic sized nucleic acid fragments may be extracted.

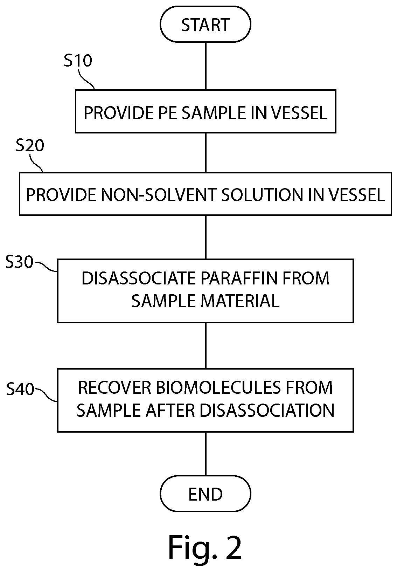

FIG. 2 shows a flow chart of steps in a method for processing a paraffin-embedded sample, such as a formalin fixed, paraffin embedded sample. In step S10, a paraffin-embedded sample is provided in a vessel. While in this embodiment, the sample is a FFPE sample, the sample need not necessarily be formalin fixed, but instead may simply be paraffin embedded, e.g., the sample may be encased in a bolus of paraffin or otherwise have paraffin attached to the sample. The sample may be any suitable type of sample, such as animal or plant tissue, whether muscle, connective tissue, bone, a seed, etc. The sample may be harvested in any suitable way, such as by surgical techniques, a biopsy needle, etc., and all or a portion of the sample may be provided in the vessel. For example, a tissue sample may be embedded in paraffin and a slice or other piece of the sample may be cut by microtome or other technique from a larger piece and placed in a vessel. In some embodiments, excess paraffin may be removed from the sample prior to placement in the vessel, e.g., excess paraffin portions may be cut or broken off prior to placement of the sample in the vessel. The sample may have any suitable size and/or shape, e.g., may be a "scroll" or relatively thin, flat piece of tissue (which may be curled or rolled, or not), and may have any suitable volume. In one illustrative embodiment, a "scroll" may have a thickness of about 5 to 25 microns, and a width and length of about 10-30mm. In some embodiments, the sample may be a "core," punched out from a larger sheet of tissue, having a suitable size, volume and thickness. In an embodiment, a "core" may have a diameter of 1-3 mm and a thickness of 1-10 mm. Of course, the sample may be arranged in other ways and have other suitable shapes, such as cylindrical, spherical, irregular, multiple separate parts, etc.

The vessel may have any suitable arrangement, shape, volume, etc., so long as the vessel is arranged to hold the sample and permit acoustic treatment of the sample suitable to disassociate paraffin from the sample material. In some embodiments, the vessel may be a glass or plastic tube with a cap or cover, and have a volume of about 10-100 microliters or more. In one embodiment, the vessel is a 6x16mm tube having a 150 microliter volume. The vessel may have a mechanism to allow control of a headspace in the vessel, e.g., a movable member may be arranged for positioning relative to an upper surface or interface of liquid in the vessel. Proper adjustment of the headspace volume and/or height may allow for more efficient acoustic processing of the sample, though is not necessarily required.

In step S20, a non-solvent liquid is provided in the vessel with the sample. The liquid may be aqueous, and may include a detergent material, such as SDS at a concentration of about 0.25% although other suitable concentrations or materials may be used. The liquid may be provided in sufficient volume (consistent with the selected volume of the vessel) to allow for proper emulsification of paraffin attached to the sample. Thus, the volume of liquid should be suitably large enough to allow for proper paraffin disassociation, and may be approximately 1 to 10 times or more the volume of the sample. Also, liquid may be provided to the vessel in an amount to control a headspace in the vessel as desired, e.g., to within about 1-2 mm of the vessel cap. For example, the liquid may be provided so that an interface of the liquid is suitably near a cap or other headspace control element of the vessel. Water provided with the liquid should preferably be distilled or otherwise suitably free of materials that might interfere with paraffin disassociation and/or subsequent processing, e.g., be molecular biology grade water. The sample and liquid may be provided at a suitable temperature, such as 5-60 degrees C (e.g., 5-10 degrees C, 15-25 degrees C).

In step S30, paraffin is disassociated from the sample by exposing the sample and liquid in the vessel to suitable acoustic energy. In one embodiment, the sample is exposed to focused acoustic energy sufficient to homogenize the sample and disassociate the paraffin from the sample. The sample may be exposed to focused acoustic energy for a time sufficient to disassociate enough paraffin from the sample to allow recovery of nucleic acid and/or proteome material from the sample, e.g., acoustic energy may be applied to the sample for about 100-200 seconds in some embodiments, such as 150 seconds. In some embodiments, the acoustic energy has a frequency of between about 100 kilohertz and about 100 megahertz and has a focal zone with a width of less than about 2 centimeters. The acoustic energy may originate from an acoustic energy source spaced from and exterior to the vessel, e.g., the acoustic energy may pass through a coupling medium to the vessel, such that at least a portion of the acoustic energy propagates exterior to the vessel. A peak incident power level of the acoustic energy may be about 175 watts, although other power levels, such as between 100 and 400 watts, may be used.

During this process, 90%, 95%, 98% or more of the paraffin attached to the sample may be disassociated and caused to be emulsified or otherwise dispersed into the liquid. In some embodiments, disassociation of the paraffin may occur at temperatures below the melting temperature of the paraffin, e.g., the vessel may be located in a bath of liquid (such as a water-based acoustic coupling medium) at a temperature of about 5-50 degrees C, such as about 7 degrees C, 20 degrees C, 40 degrees C or 46 degrees C. Thus, in some examples, the paraffin may be disassociated from the sample while the sample remains below a temperature of about 55 degrees C. In some embodiments, the average particles size of the paraffin after emulsification is between 0.1-10 microns.

In step S40, biomolecules of the sample may be recovered from the vessel that are essentially free of paraffin. This recovery may be performed in any suitable way, such as by physically grasping material in the vessel and removing the material, pipetting liquid and sample material from the vessel, filtering the sample material from the liquid, centrifuging the vessel to separate sample material from paraffin and/or other material in the vessel, and so on. As discussed above, additional processes may be involved in the recovery of biomolecules from the sample, including digesting proteins or other substances in the sample, shearing or fragmenting nucleic acids, additional acoustic treatment after paraffin disassociation, adding reagents or other substances to the vessel, and so on.

For example, a protease may be added to the non-solvent aqueous solution and the sample in the vessel after disassociation of paraffin from the sample. In some embodiments, the protease, which may be proteinase K or trypsin, may be added to the vessel without removing the disassociated paraffin. Also, or alternately, after addition of the proteinase K or trypsin, the sample may be again exposed to acoustic energy, e.g., focused acoustic energy, arranged to mix the sample and protease, and thereby enhance activity of the proteinase K or trypsin. For example, the sample and proteinase K or trypsin may be exposed to 10-30 seconds (or more) of focused acoustic energy to mix the protease and sample. After acoustic treatment, the sample may be incubated for a suitable period of time (e.g., between 15 minutes to 1 hour, or over an hour) with the proteinase K or trypsin such as at about 56 degrees C for proteinase K. In some embodiments, incubation of a sample of DNA with proteinase K or trypsin occurs for approximately 1 hour. In some embodiments, incubation of a sample of RNA with proteinase K or trypsin occurs for approximately 15 minutes.

As discussed, the sample may be subject to any suitable enzyme treatment after paraffin disassociation. For example, when processing a sample of DNA, in addition to protease treatment, the sample may be subject to RNase digestion, to remove residual RNA; and conversely, when processing a sample of RNA, the sample may be subject to DNase digestion, to remove residual DNA.

An additional incubation of the sample may be done for a suitable period, e.g., extended for 1 hour or more or for less than 1 hour (e.g., 15 minutes), at a suitable temperature, such as about 80 or 90 degrees C to reverse crosslinks in the sample material. In some embodiments, reverse crosslinking of a DNA sample at a temperature between about 80-90 C may occur for approximately 1 hour. In some embodiments, reverse crosslinking of a RNA sample at a temperature between about 80-90 C may occur for approximately 15 minutes.

If nucleic acids in the sample are to be fragmented and recovered from the sample, the sample (e.g., in the same vessel and liquid used in paraffin disassociation and/or protease digestion, or in another vessel) may be subjected to acoustic processing to shear DNA or RNA fragments into a desired size range. For example, as discussed above, after exposing the sample to an appropriate amount of focused acoustic energy, nucleic acid material may be fragmented such that a majority of the fragments have a size of 50 to 500 bp. As also discussed above, the sample may be exposed to a short burst of focused acoustic energy, resulting in slight fragmentation of the nucleic acids (e.g., 1500-5000 bp), yet higher recovery than if the sample were exposed to focused acoustic energy for a longer period of time, or if no focused acoustic energy were applied at all.

Thereafter, the fragmented nucleic acids may be recovered, e.g., by centrifuging the vessel (or its contents) and pipetting the nucleic acids from the vessel, leaving the paraffin (if present) and/or other materials in the vessel. The recovered nucleic acids present in the aqueous phase of the sample may be then purified or otherwise processed, e.g., using known techniques for purification. For example, the sample may be added to a chaotropic salt solution, usually containing guanidine, and ethanol may be added to the nucleic acid material and mixed by vortexing. The volumes of salt solution and ethanol used will typically be scaled to the liquid volume of the sample. After mixing by vortexing, the vessel may be centrifuged (e.g., at 10,000xg for 2 minutes) at room temperature or other suitable temperature. After centrifugation, paraffin particles, if present in the vessel, will form a white, floating layer on top of the liquid. The vessel may be held at the same angle as in the centrifuge rotor, and a pipet used to recover the nucleic acid-containing liquid from the vessel.

The liquid may then be transferred to a suitable purification column (e.g., silica membrane column) with a binding buffer, and subsequently eluted in water. Generally, any transfer of some paraffin particles from the vessel is acceptable and will not interfere with the nucleic acid purification.

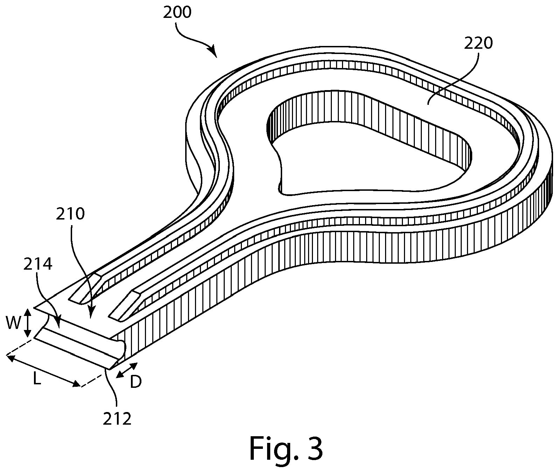

In addition, in some instances, a tool may be used to collect tissue sections from microscopic slides, for subsequent processing. FIG. 3 illustrates an illustrative embodiment of a collecting tool 200 having a collection portion 210, for engaging with a tissue sample (e.g., FFPE sample) resting on a slide; and a handle 220, for suitably manipulating the tool. For instance, the collecting tool 200 can be appropriately pushed along the surface of a microscope slide so as to scrape off and remove the tissue sample from the slide.

During use, a user may position the collecting tool 200 such that the edge 212 of the collection portion rests adjacent to the tissue sample resting on the surface of the slide. The user may then grip the handle 220 and push the tool along the slide surface so that the relatively sharp edge 212 becomes wedged underneath the sample, lifting the sample up from the surface of the slide and into the recess 214. As more sample is lifted from the slide and collects within the recess 214, the sample conforms into the overall shape dictated by the recess. For example, as the tissue sample collects into the recess 214, the sample folds into a rod-like shape.

Upon suitable collection, the sample is easily transferred, for example, into a container/tube for processing/analysis. That is, the user may invert the tool over the opening of the processing vessel such that the folded up tissue sample is able to slide therein.

The recess 214 may have any suitable dimensions of length L, width W and depth D. In some embodiments, the recess has a length L of between 1 mm and 50 mm, or between 1 mm and 30 mm (e.g., 5-20 mm). In some embodiments, the recess has a width W of between 0.5 mm and 10 mm, or between 1 mm and 5 mm (e.g., 1-3 mm). In some embodiments, the recess has a depth D of between 0.5 mm and 10 mm, or between 1 mm and 5 mm (e.g., 1-3 mm). It can be appreciated that the dimensions of the recess may fall within other appropriate ranges.

The collecting tool may include any suitable material. In some embodiments, the tool is made up of a medical grade material, such as a medical device grade polycarbonate. For example, Makrolon 2458 may be a suitable material used to form the tool. Such a material may have a relatively low viscosity, so as to maintain sharpness of the edge (e.g., upon heating). The material may also have a suitable hardness, for instance, a Rockwell hardness of 75 M scale. Accordingly, the tool may be appropriate for numerous uses in prying a sample, without damage, up off of a slide on which the sample may otherwise be adhered.

As the collecting tool may be used to handle biological specimens, the tool itself may be suitable for regular cleaning, for example, by autoclave, ethylene oxide (EtO) and/or steam sterilization. The tool may be cleaned or sterilized according to other methods as well.

In some embodiments, the collecting tool 200 is used in cooperation with a slide warmer that elevates the temperature of the slide. As such, the slide warmer may cause the tissue sample to be more easily separated from the slide than if the temperature were lower.

As described above, the system control circuit 10 may include any suitable components to perform desired control, communication and/or other functions. For example, the system control circuit 10 may include one or more general purpose computers, a network of computers, one or more microprocessors, etc. for performing data processing functions, one or more memories for storing data and/or operating instructions (e.g., including volatile and/or non-volatile memories such as optical disks and disk drives, semiconductor memory, magnetic tape or disk memories, and so on), communication buses or other communication devices for wired or wireless communication (e.g., including various wires, switches, connectors, Ethernet communication devices, WLAN communication devices, and so on), software or other computer-executable instructions (e.g., including instructions for carrying out functions related to controlling the load current control circuit as described above and other components), a power supply or other power source (such as a plug for mating with an electrical outlet, batteries, transformers, etc.), relays and/or other switching devices, mechanical linkages, one or more sensors or data input devices (such as a sensor to detect a temperature and/or presence of the medium 16, a video camera or other imaging device to capture and analyze image information regarding the vessel 4 or other components, position sensors to indicate positions of the acoustic transducer 14 and/or the vessel 4, and so on), user data input devices (such as buttons, dials, knobs, a keyboard, a touch screen or other), information display devices (such as an LCD display, indicator lights, a printer, etc.), and/or other components for providing desired input/output and control functions.

### Example 1

A comparison test was performed to compare nucleic acid (DNA in this case) yield from an FFPE sample using processes in accordance with aspects of the invention with the yield obtained using a Promega ReliaPrep FFPE gDNA Miniprep system.

A buffer containing 0.25% of SDS was used to disassociate paraffin from the sample. 106 microliters of this buffer was loaded into a Covaris microTUBE Screw-Cap vessel. Two pieces of scroll, 20micrometers in thickness and a weight of less than 5mg were loaded in the same microTUBE vessel.

A first acoustic treatment (with a Covaris E or S-Series) consisting of 175W , 10% duty factor, 150 seconds and 200cycles per burst at 41 degrees C was used to disassociate the paraffin from the sample and rehydrate it. 20 microliters of Proteinase K (at 20mg/ml) was then added in the same microTUBE vessel. A short acoustic treatment (175W / 10% duty factor / 10 seconds / 200 cycles per burst) was then done to mix the Proteinase K and the sample.

The sample was then incubated at 56 degrees C for an hour to let the Proteinase K digest the tissue. Another incubation step of an hour at 80 degrees C was then realized to reverse the crosslink between nucleic acids and proteins.

At the end, using the same microTUBE vessel and without transferring the sample or paraffin, an acoustic treatment (175W / 10 % duty factor / 200 cycles per burst / 430 seconds) was done to fragment the DNA to a size suitable for next gen sequencing. The acoustic treatment reduced the fragment size to a distribution centered on 200bp and comprised between 50 and 500 bp.

In short, when the DNA fragments were purified and quantitated from the above example, the yield was 2 to 3 times the yield obtained with the Promega system.

### Example 2

A test similar to that described in Example 1 was performed to compare RNA yield from 10 micron sections obtained from an FFPE kidney tissue sample, using processes in accordance with aspects of the invention with the yield obtained using a conventional PROMEGA ReliaPrep FFPE Total RNA Miniprep System.

The protocol for RNA preparation for the FFPE sample was essentially the same as that described above for DNA in Example 1, except certain processing conditions were varied. In this protocol, the RNA sample was incubated with Proteinase K at 56 degrees C for 15 minutes, allowing for protein digestion. The sample was further incubated at 80 degrees C for 15 minutes, allowing for crosslink reversal between RNA and proteins. The sample was then exposed to DNase to remove any residual DNA. The RNA was then bound in a purification column and washed with a binding buffer. RNA molecules were subsequently eluted in 50 microliters of water.

The RNA fragments were purified and quantified, using a QUBIT RNA HS Assay Kit. It was found that the RNA recovery from two separate samples that were processed using focused acoustic energy were 125% and 144% in yield as compared to two comparative samples processed using the PROMEGA system.

### Example 3

DNA was purified and recovered from FFPE samples using a Covaris FFPE DNA Purification Kit and tested by a multiplex PCR-based quality control assay, for quantitative PCR (qPCR). The purified DNA was recovered at yields of greater than 2-4 times than that from using the QIAGEN QIAamp DNA FFPE Tissue Kit, which employs a solvent-based method of paraffin removal.

A microtome was used to cut FFPE tissue sections approximately 7 to 25 microns in thickness, from which FFPE tissue cores approximately 1.2 mm in diameter and approximately 12 mg were punched. The FFPE tissue cores were loaded with SDS buffer into a Covaris microTUBE Screw-Cap vessel.

A first focused acoustic treatment (with a Covaris E or S-Series) consisting of 175W , 10% duty factor, 300 seconds and 200 cycles per burst at 20 degrees C was used to disassociate the paraffin from the sample while simultaneously rehydrating the tissue. During the focused acoustic treatment, the sample became emulsified, gaining a milky white appearance.

Then, 20 microliters of Proteinase K was added to the sample within the microTUBE vessel. A short focused acoustic treatment (using a Covaris S220 or E220 system with the following parameters: 175W / 10% duty factor / 10 seconds / 200 cycles per burst) at 20 degrees C was then performed so as to mix the Proteinase K and the sample together.

The sample was then incubated at 56 degrees C for an hour to let the Proteinase K digest the tissue. The sample further incubated for an hour at 80 degrees C to reverse crosslinks between nucleic acids and proteins.

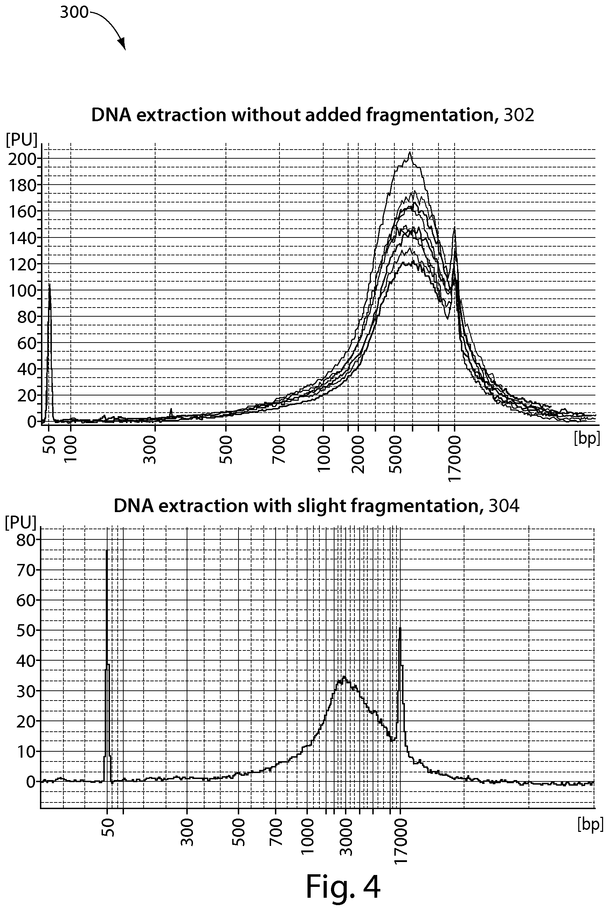

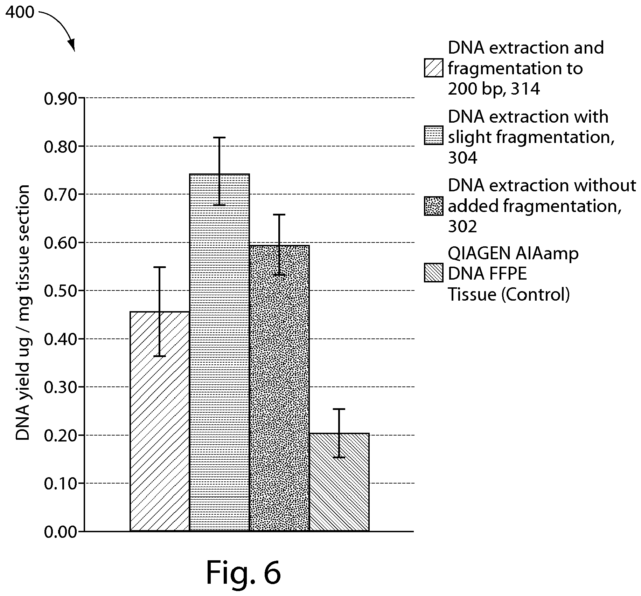

After crosslink reversal, samples were processed according to three different options. Figs. 4-5 show fragment size distributions 300, 310 according to qPCR for DNA samples processed according to each of the three different options. Fig. 6 illustrates the amount of DNA recovery from the respective samples, as well as DNA recovery from the QIAGEN QIAamp DNA FFPE Tissue Kit.

In the first processing option, the DNA sample was purified without further acoustic treatment and the sample was analyzed according to qPCR. Because the DNA was not exposed to any additional focused acoustic treatment, and thus not fragmented, the size of the DNA fragments were relatively large as compared to DNA samples that were subject to additional focused acoustic treatment. Fig. 4 shows the DNA fragment size distribution 302 for samples processed according to the first option. As shown, the distribution of DNA fragment size has a peak at over 5000 bp. Fig. 6 shows that the amount of DNA recovery (i.e., approximately 0.60 µg/mg tissue section) was significant, comparatively greater (about 3x) than the amount of DNA recovery (i.e., approximately 0.20 µg/mg tissue section) from usage of the QIAGEN QIAamp DNA FFPE Tissue Kit.

In the second processing option, the DNA sample was purified and subject to a short burst of acoustic energy, using Covaris S220 or E220 system with the following parameters: 105W / 10% duty factor / 10 seconds / 200 cycles per burst, at 20 degrees C within the microTUBE. In this case, the additional focused acoustic treatment resulted in a slight degree of DNA fragmentation, yet also enhanced the overall DNA recovery. Fig. 4 depicts the DNA fragment size distribution 304 for samples processed according to the second option, showing a distribution peak to be over 2000 bp. Fig. 6 shows that there was even more DNA recovery (i.e., approximately 0.75 µg/mg tissue section) than that observed using the first processing option where the sample was not exposed to additional focused acoustic treatment. The DNA recovery resulting from the short burst of focused acoustics was almost 4x greater than the amount of DNA recovery (i.e., approximately 0.20 µg/mg tissue section) from usage of the QIAGEN QIAamp DNA FFPE Tissue Kit.

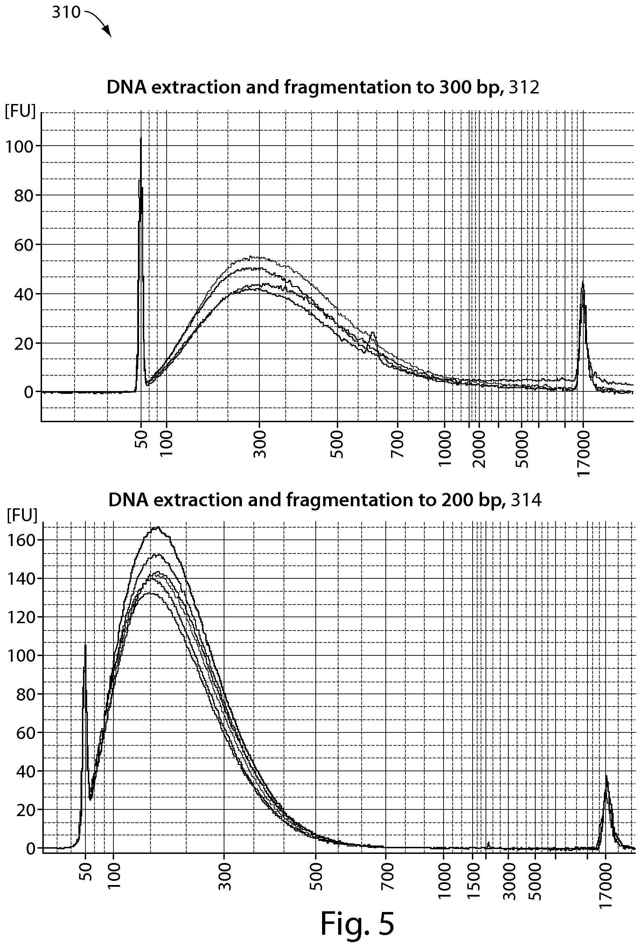

In the third processing option, the DNA sample was purified and fragmented using focused acoustics, to a desired fragment size. Using the Covaris S220 or E220 system with the following parameters: 175W / 10% duty factor / 200 cycles per burst at 20 degrees C within the microTUBE, the treatment time would vary depending on the desired fragment size. That is, to obtain a DNA fragment size distribution having a peak at approximately 300 bp, as shown by the distribution 312 in Fig. 5, the focused acoustic treatment would last for approximately 110 seconds; and to obtain a DNA fragment size distribution having a peak at approximately 200 bp, as shown by the distribution 314 in Fig. 5, the focused acoustic treatment would last for approximately 300 seconds. Fig. 6 shows the DNA recovery (i.e., approximately 0.45 µg/mg tissue section) after fragmentation to be more than 2x the greater than the amount of DNA recovery (i.e., approximately 0.20 µg/mg tissue section) from usage of the QIAGEN QIAamp DNA FFPE Tissue Kit.

In summary, when the DNA fragments were purified, optionally fragmented, and quantified using methods according to the above example, the yield was much greater (e.g., 2-4 times) than the yield obtained with the QIAGEN system.

### Example 4

In another example, fluorescent microscopy was used to study paraffin disassociation from FFPE tissue, via focused acoustics. FFPE tissue samples obtained from normal human kidneys were provided by the Cooperative Human Tissue Network, Eastern Division University of Pennsylvania. A microtome was used to cut FFPE samples approximately 25 microns thick.

FFPE tissue sections were placed into a Covaris screw-cap microTUBE, along with 110 microliters of SDS Buffer. The microTUBE containing the sample was loaded into a Covaris S220 Focused-ultrasonicator and processed with focused acoustic energy, according to the following parameters: 10% Duty Factor, 175 W PIP, 200 cycles per burst, for 5 minutes at 20 degrees C. The focused acoustic energy served to disassociate the paraffin from the tissue sample while simultaneously rehydrating the tissue. During the focused acoustic process, the sample was emulsified, gaining a milky white appearance.

The tissue sample was spun transferred to a 1.5 mL microcentrifuge tube and washed three times with 1 mL water, removing emulsified wax particles.

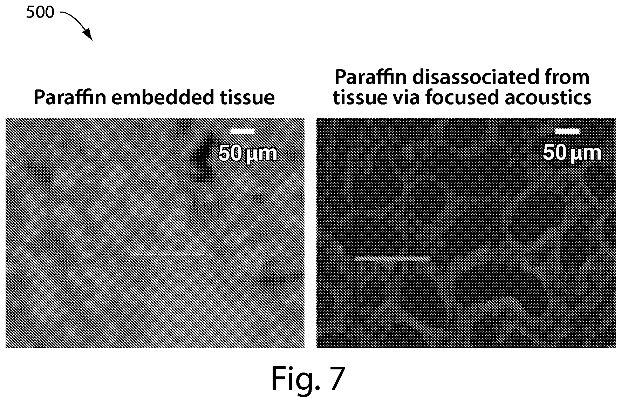

After washing away of lose paraffin, samples were mounted on a microscope slide and dried for at least 30 minutes. A fluorescent microscope (Olympus Model IX73 with Fluorescence and EXI-BLU Camera) was used to study auto-fluorescence of the paraffin remaining in the tissues after the removal treatment. Fig. 7 shows fluorescence images 500 of paraffin embedded tissue and a tissue sample processed using focused acoustics, where the paraffin was disassociated therefrom.

Focused acoustic treatment was observed to provide more efficient and effective paraffin disassociation from the tissue samples than that observed from the QIAGEN FFPE Tissue Kit. On average, the auto-fluorescence intensity of left-over paraffin in the tissue was three times lower and more homogeneous for samples treated with the Covaris system as compared with samples treated with the QIAGEN FFPE Tissue Kit. For instance, fluorescent intensity was observed to vary up to 40% in samples treated with the QIAGEN FFPE Tissue Kit. In contrast, fluorescent intensity of samples treated with focused acoustics was observed to be substantially more stable, with less than a 20% variation. Homogenous disassociation of paraffin from the tissue sample allows for more efficient tissue rehydration (e.g., enzymes such as protease will only digest hydrated portions of the tissue), allowing for unbiased digestion and extraction of nucleic acids representative of the entire sample.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

The use of "including," "comprising," "having," "containing," "involving," and/or variations thereof herein, is meant to encompass the items listed thereafter and equivalents thereof as well as additional items.

It should also be understood that, unless clearly indicated to the contrary, in any methods claimed herein that include more than one step or act, the order of the steps or acts of the method is not necessarily limited to the order in which the steps or acts of the method are recited.

## Claims

1. A method for processing a paraffin-embedded sample, comprising:
providing a paraffin-embedded tissue sample in a vessel, the sample having previously been formalin fixed and embedded in paraffin;
providing a solution in the vessel with the paraffin-embedded sample, wherein the solution is a non-solvent solution for the paraffin;
disassociating paraffin from the paraffin-embedded sample by exposing the sample and non-solvent solution in the vessel to acoustic energy having a frequency of between 100 kilohertz and 100 megahertz and a focal zone with a width of less than 2 centimeters to disassociate paraffin from the sample; and
recovering biomolecules from the sample after disassociation of paraffin from the sample.

2. The method of claim 1, wherein DNA, RNA and/or protein is recovered from the sample after the disassociation of paraffin.

3. The method of claim 1, wherein the step of disassociating comprises disassociating more than 90% of paraffin attached to the sample.

4. The method of claim 1, wherein the step of disassociating comprises rehydrating the sample while exposing the sample to focused acoustic energy.

5. The method of claim 1, wherein the step of disassociating comprises exposing the sample to focused acoustic energy while the vessel is located in a bath of liquid at a temperature of 5-60 degrees C.

6. The method of claim 1, wherein the step of disassociating comprises maintaining a temperature of the sample below 60 degrees C.

7. The method of claim 1, wherein the sample has a thickness of 5 to 25 microns and a length of less than 25 mm.

8. The method of claim 1, wherein the sample has a diameter of 1 to 3 mm and a thickness of 1 to 10 mm.

9. The method of claim 1, further comprising adding a protease to the non-solvent solution and the sample in the vessel after disassociation of paraffin from the sample.

10. The method of claim 9, wherein the step of exposing the sample and the protease-containing solution to focused acoustic energy comprises exposing the sample to focused acoustic energy suitable to fragment nucleic acid material from the sample into smaller fragments.

11. The method of claim 9, further comprising incubating the sample in the vessel with the protease-containing solution at a temperature of 80°C to 90°C to reverse formaldehyde crosslinks in the sample.

12. The method of claim 11, wherein the sample comprises DNA and the step of exposing the sample and the protease-containing solution to additional focused acoustic energy results in enhanced DNA recovery and fragmenting of the DNA to fragments larger than 2.0 kbp.

13. The method of claim 1, wherein the recovering step includes centrifuging the vessel containing the sample, disassociated paraffin and non-solvent solution of nucleic acid and/or proteome material to transfer the contents from the vessel to a second container.

14. The method of claim 1, wherein the focal zone has a width of less than 2 centimeters, and the acoustic energy originates from an acoustic energy source spaced from and exterior to the vessel, wherein at least a portion of the acoustic energy propagates exterior to the vessel.

## Patentansprüche

1. Verfahren zum Verarbeiten einer in Paraffin eingebetteten Probe, umfassend:
Bereitstellen einer in Paraffin eingebetteten Gewebeprobe in einem Gefäß, wobei die Probe zuvor Formalinfixiert und in Paraffin eingebettet wurde;
Bereitstellen einer Lösung in dem Gefäß mit der in Paraffin eingebetteten Probe, wobei die Lösung eine Nicht-Lösungsmittel-Lösung für das Paraffin ist;
Trennen von Paraffin aus der in Paraffin eingebetteten Probe, indem die Probe und die Nicht-Lösungsmittel-Lösung in dem Gefäß akustischer Energie mit einer Frequenz zwischen 100 Kilohertz und 100 Megahertz und einer Fokuszone mit einer Breite von weniger als 2 Zentimeter ausgesetzt wird und so Paraffin von der Probe getrennt wird; und
Rückgewinnen von Biomolekülen aus der Probe nach dem Trennen von Paraffin aus der Probe.

2. Verfahren nach Anspruch 1, bei dem DNA, RNA und/oder Protein aus der Probe nach dem Trennen von Paraffin rückgewonnen wird.

3. Verfahren nach Anspruch 1, bei dem der Trennungsschritt das Trennen von mehr als 90% des an die Probe gebundenen Paraffins umfasst.

4. Verfahren nach Anspruch 1, bei dem der Trennungsschritt das Rehydrieren der Probe, während die Probe fokussierter akustischer Energie ausgesetzt wird, umfasst.

5. Verfahren nach Anspruch 1, bei dem der Trennungsschritt umfasst, dass die Probe fokussierter akustischer Energie ausgesetzt wird, während das Gefäß sich in einem Flüssigkeitsbad bei einer Temperatur von 5-60 °C befindet.

6. Verfahren nach Anspruch 1, bei dem der Trennungsschritt das Halten einer Temperatur der Probe unter 60 °C umfasst.

7. Verfahren nach Anspruch 1, bei dem die Probe eine Dicke von 5 bis 25 Mikrometer und eine Länge von weniger als 25 mm aufweist.

8. Verfahren nach Anspruch 1, bei dem die Probe einen Durchmesser von 1 bis 3 mm und Dicke von 1 bis 10 mm aufweist.

9. Verfahren nach Anspruch 1, das ferner das Hinzufügen einer Protease zu der Nicht-Lösungsmittel-Lösung und der Probe in dem Gefäß nach dem Trennen von Paraffin aus der Probe umfasst.

10. Verfahren nach Anspruch 9, bei dem der Schritt, in dem die Probe und die Protease-enthaltende Lösung fokussierter akustischer Energie ausgesetzt wird, umfasst, dass die Probe fokussierter akustischer Energie ausgesetzt wird, die dazu geeignet ist Nukleinsäurematerial aus der Probe in kleinere Fragmente zu fragmentieren.

11. Verfahren nach Anspruch 9, das ferner das Inkubieren der Probe in dem Gefäß mit der Protease-enthaltenen Lösung bei einer Temperatur von 80 °C bis 90 °C, um Formaldehydvernetzungen in der Probe rückgängig zu machen, umfasst.

12. Verfahren nach Anspruch 11, bei dem die Probe DNA umfasst und der Schritt, in dem die Probe und die Protease-enthaltende Lösung zusätzlicher fokussierter akustischer Energie ausgesetzt wird, zu verbesserter DNA-Rückgewinnung und Fragmentierung der DNA in Fragmente, die größer als 2,0 kbp sind, führt.

13. Verfahren nach Anspruch 1, bei dem der Rückgewinnungsschritt das Zentrifugieren des Gefäßes, das die Probe, getrenntes Paraffin und Nicht-Lösungsmittel-Lösung der Nukleinsäure und/oder des Proteommaterials enthält, einschließt, und so die Inhalte von dem Gefäß in einen zweiten Behälter überführt werden.

14. Verfahren nach Anspruch 1, bei dem die Fokuszone eine Breite von weniger als 2 Zentimeter aufweist und die akustische Energie von einer akustischen Energiequelle stammt, die mit Abstand von und außerhalb des Gefäßes angeordnet ist, wobei sich mindestens ein Teil der akustischen Energie außerhalb des Gefäßes ausbreitet.

## Revendications

1. Procédé pour traiter un échantillon enrobé de paraffine, comprenant le fait de :
fournir un échantillon de tissu enrobé de paraffine dans un récipient, l'échantillon ayant été fixé au préalable avec de la formaline et ayant été enrobé dans de la paraffine ;
fournir une solution dans le récipient avec l'échantillon enrobé de paraffine, dans laquelle la solution est une solution exempte de solvant pour la paraffine ;
dissocier la paraffine de l'échantillon paraffine en exposant l'échantillon et la solution exempte de solvant dans le récipient à une énergie acoustique possédant une fréquence entre 100 kHz et 100 MHz et une zone focale dont la largeur est inférieure à 2 cm afin de dissocier la paraffine de l'échantillon ; et
récupérer des biomolécules de l'échantillon après dissociation de la paraffine par rapport à l'échantillon.

2. Procédé selon la revendication 1, dans lequel de l'ADN, de l'ARN et/ou des protéines sont récupérés de l'échantillon après la dissociation de la paraffine.

3. Procédé selon la revendication 1, dans lequel l'étape de dissociation comprend une dissociation de plus de 90 % de la paraffine fixée à l'échantillon.

4. Procédé selon la revendication 1, dans lequel l'étape de dissociation comprend la réhydratation de l'échantillon lors de l'exposition de l'échantillon à de l'énergie acoustique focalisée.

5. Procédé selon la revendication 1, dans lequel l'étape de dissociation comprend l'exposition de l'échantillon à de l'énergie acoustique focalisée tandis que le récipient est situé dans un bain de liquide à une température de 5 à 60 °C.

6. Procédé selon la revendication 1, dans lequel l'étape de dissociation comprend le maintien d'une température de l'échantillon inférieure à 60 °C.

7. Procédé selon la revendication 1, dans lequel l'échantillon possède une épaisseur de 5 à 25 microns et une longueur inférieure à 25 mm.

8. Procédé selon la revendication 1, dans lequel l'échantillon possède un diamètre de 1 à 3 mm et une épaisseur de 1 à 10 mm.

9. Procédé selon la revendication 1, comprenant en outre l'addition d'une protéase à la solution exempte de solvant et à l'échantillon dans le récipient après dissociation de la paraffine par rapport à l'échantillon.

10. Procédé selon la revendication 9, dans lequel l'étape d'exposition de l'échantillon et de la solution contenant une protéase à de l'énergie acoustique focalisée comprend l'exposition de l'échantillon à de l'énergie acoustique focalisée appropriée pour fragmenter la matière d'acide nucléique provenant de l'échantillon en fragments plus petits.

11. Procédé selon la revendication 9, comprenant en outre l'incubation de l'échantillon dans le récipient avec la solution contenant une protéase à une température de 80 °C à 90 °C afin de renverser les réticulations de formaldéhyde dans l'échantillon.

12. Procédé selon la revendication 11, dans lequel l'échantillon comprend de l'ADN, et l'étape d'exposition de l'échantillon et de la solution contenant une protéase à une énergie acoustique focalisée supplémentaire donne lieu à une récupération augmentée d'ADN et à une fragmentation de l'ADN en fragments plus grands que 2,0 kpb.

13. Procédé selon la revendication 1, dans lequel l'étape de récupération comprend une centrifugation du récipient contenant l'échantillon, la paraffine dissociée et la solution d'acide nucléique exempte de solvant et/ou une matière protéomique afin de transférer le contenu du récipient à un second contenant.

14. Procédé selon la revendication 1, dans lequel la zone focale possède une largeur inférieure à 2 cm, et l'énergie acoustique émane d'une source d'énergie acoustique espacée du récipient et à l'extérieur de celui-ci, dans lequel au moins une portion de l'énergie acoustique se propage à l'extérieur du récipient.
